(19) Europäisches Patentamt
European Patent Office
Office européen des brevets

(11) Veröffentlichungsnummer : **0 024 538**
**B1**

(12) # EUROPÄISCHE PATENTSCHRIFT

(45) Veröffentlichungstag der Patentschrift : **26.01.83**

(51) Int. Cl.³ : **C 07 D249/08**, A 01 N 43/64 // (C07D249/08, C07C49/175)

(21) Anmeldenummer : **80104236.7**

(22) Anmeldetag : **18.07.80**

(54) **1,2,4-Triazolyl-enolether, Verfahren zu ihrer Herstellung und ihre Verwendung als Fungizide.**

(30) Priorität : **04.08.79 DE 2931755**

(43) Veröffentlichungstag der Anmeldung : **11.03.81 Patentblatt 81/10**

(45) Bekanntmachung des Hinweises auf die Patenterteilung : **26.01.83 Patentblatt 83/04**

(84) Benannte Vertragsstaaten : **AT BE CH DE FR GB IT LI NL SE**

(56) Entgegenhaltungen :
**DE A 2 600 799**
**DE A 2 720 868**
**DE A 2 720 949**

(73) Patentinhaber : **BAYER AG**
**Zentralbereich Patente, Marken und Lizenzen**
**D-5090 Leverkusen 1, Bayerwerk (DE)**

(72) Erfinder : **Draber, Wilfried, Dr.**
**In den Birken 81**
**D-5600 Wuppertal 1 (DE)**
Erfinder : **Büchel, Karl Heinz, Dr.**
**Haus an der Dachsdelle Dabringhauser Strasse 42**
**D-5093 Burscheid (DE)**
Erfinder : **Frohberger, Paul-Ernst, Dr.**
**Willi-Baumeister-Strasse 5**
**D-5090 Leverkusen (DE)**
Erfinder : **Brandes, Wilhelm, Dr.**
**Eichendorffstrasse 3**
**D-5623 Leichlingen (DE)**

**EP 0 024 538 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

1,2,4-Triazolyl-enolether, Verfahren zu ihrer Herstellung und ihre Verwendung als Fungizide

Die Erfindung betrifft neue 1,2,4-Triazolylenolether, ein Verfahren zu ihrer Herstellung und ihre Verwendung als Fungizide.

Es ist bereits bekannt geworden, daß 1,2,4-Triazolylether, wie beispielsweise 1-(4-Chlorphenoxy)-2-(2,4-dichlorbenzyloxy)-3,3-dimethyl-1-(1,2,4-triazol-1-yl)-butan, sowie 1,2,4-Triazolyl-pentanone, wie beispielsweise 1-(4-Chlorphenyl)-4,4-dimethyl-2-(1,2,4-triazol-1-yl)-3-pentanon gute fungizide Eigenschaften aufweisen (vergleiche DE-OS 27 20 949 sowie DE-OS 27 34 426). Die Wirkung dieser Verbindungen ist jedoch, insbesondere bei niedrigen Aufwandmengen und -konzentrationen, nicht immer ganz befriedigend.

Es wurden neue 1,2,4-Triazolyl-enolether der allgemeinen Formel I

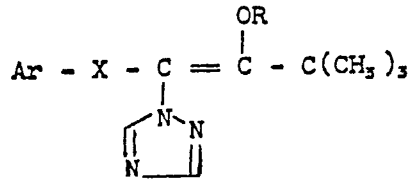

(I)

in welcher

Ar für Phenyl und Naphthyl steht, wobei diese Reste substituiert sein können durch Halogen, Alkyl mit 1 bis 6 und Alkoxy mit 1 bis 4 Kohlenstoffatomen, Halogenalkyl mit 1 bis 4 Kohlenstoff- und 1 bis 5 Halogenatomen, ferner durch Nitro, Cyano und schließlich durch gegebenenfalls halogensubstituiertes Phenyl,

R für Alkyl mit 1 bis 4 Kohlenstoffatomen steht und

X für Sauerstoff oder für die Methylengruppe steht, gefunden, sowie deren Säure- und Metallsalz-Additionsverbindungen.

Die erfindungsgemäßen Verbindungen der allgemeinen Formel I kommen in den geometrischen Isomeren E(trans) und Z(cis) vor. Bei der E, Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, ist die Konfiguration Z (abgeleitet von zusammen), stehen sie auf entgegengesetzter Seite, E (abgeleitet von entgegen). Sowohl die einzelnen Isomeren als auch die Gemische werden erfindungsgemäß beansprucht.

Weiterhin wurde gefunden, daß man die 1,2,4-Triazolylenolether der allgemeinen Formel I erhält, wenn man 1,2,4-Triazolyl-ketone der allgemeinen Formel II

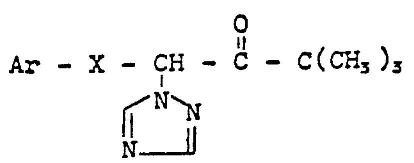

(II)

in welcher

Ar und X die oben angegebene Bedeutung haben, mit entsprechenden Alkylsulfaten bzw. -halogeniden in Gegenwart einer Base und in Gegenwart eines organischen Verdünnungsmittels, oder in einem wässrig-organischen Zweiphasensystem in Gegenwart eines Phasentransferkatalysators umsetzt, und gegebenenfalls anschließend eine Säure oder ein Metallsalz addiert.

Die neuen 1,2,4-Triazolyl-enolether der allgemeinen Formel I weisen starke fungizide Eigenschaften auf. Dabei zeigen überraschenderweise die erfindungsgemäßen Verbindungen eine erheblich höhere Wirkung als die aus dem Stand der Technik bekannten Verbindungen 1-(4-Chlorphenoxy)-2-(2,4-dichlorbenzyloxy)-3,3-dimethyl-1-(1,2,4-triazol-1-yl)-butan und 1-(4-Chlorphenyl)-4,4-dimethyl-2-(1,2,4-triazol-1-yl)-3-pentanon, welches chemisch und wirkungsmäßig naheliegende Verbindungen sind. Die erfindungsgemäßen Stoffe stellen somit eine Bereicherung der Technik dar.

Die erfindungsgemäßen 1,2,4-Triazolyl-enolether sind durch die allgemeine Formel I definiert. In dieser Formel steht Ar für gegebenenfalls einfach oder mehrfach, gleich oder verschieden substituiertes Phenyl und Naphthyl, wobei als Substituenten in Frage kommen : Halogen ; Alkyl mit insbesondere 1 bis 4 Kohlenstoffatomen ; Alkoxy mit insbesondere 1 bis 2 Kohlenstoffatomen ; Halogenalkyl mit insbesondere bis zu 2 Kohlenstoff- und bis zu drei gleichen oder verschiedenen Halogenatomen, wobei als Halogene insbesondere Fluor und Chlor stehen ; Nitro ; Cyano ; sowie gegebenenfalls durch Halogen, insbesondere Chlor substituiertes Phenyl. R steht für geradkettiges oder verzweigtes Alkyl mit 1 bis 4 Kohlenstoffatomen und X steht für Sauerstoff und die Methylengruppe.

Besonders bevorzugt sind diejenigen 1,2,4-Triazolyl-enolether der allgemeinen Formel I, in denen Ar für Phenyl steht, das gegebenenfalls einfach oder zweifach, gleich oder verschieden substituiert ist durch

Fluor, Chlor, Brom, Jod, Methyl, Ethyl, Isopropyl, Trifluormethyl, Nitro, Phenyl oder Chlorphenyl ; R für Methyl, Ethyl, Isopropyl, Isobutyl oder tert.-Butyl steht ; und X die in der Erfindungsdefinition angegebene Bedeutung hat.

Im einzelnen seien außer den bei den Herstellungsbeispielen genannten Verbindungen die folgenden Verbindungen der allgemeinen Formel I genannt :

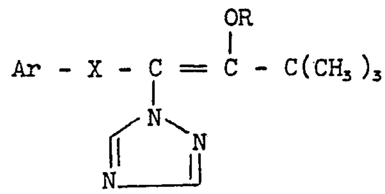 (I)

| Ar | X | R |
|---|---|---|
| 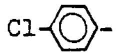 | O | $i\text{-}C_4H_9$ |
| 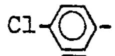 | $CH_2$ | $CH_3$ |
| 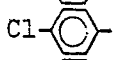 | $CH_2$ | $i\text{-}C_3H_7$ |
| 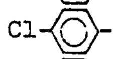 | $CH_2$ | $i\text{-}C_4H_9$ |
| 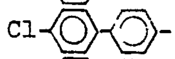 | O | $CH_3$ |
| 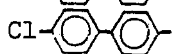 | O | $C_2H_5$ |
| 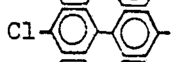 | $CH_2$ | $CH_3$ |
| 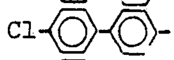 | $CH_2$ | $C_2H_5$ |
| 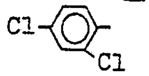 | O | $CH_3$ |
| 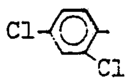 | O | $C_2H_5$ |
| 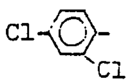 | $CH_2$ | $CH_3$ |
| 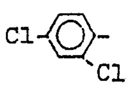 | $CH_2$ | $C_2H_5$ |
| 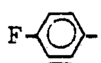 | O | $CH_3$ |
| 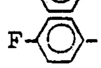 | O | $C_2H_5$ |
| 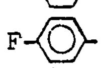 | $CH_2$ | $CH_3$ |
| 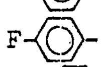 | $CH_2$ | $C_2H_5$ |
| 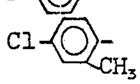 | O | $CH_3$ |
| 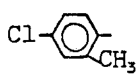 | O | $C_2H_5$ |
| 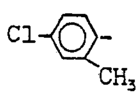 | $CH_2$ | $CH_3$ |
| 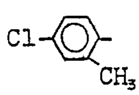 | $CH_2$ | $C_2H_5$ |

| Ar | X | R |
|---|---|---|
| 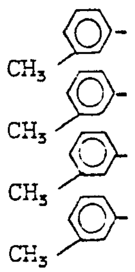 | O | $CH_3$ |
| | O | $C_2H_5$ |
| | $CH_2$ | $CH_3$ |
| | $CH_2$ | $C_2H_5$ |
| 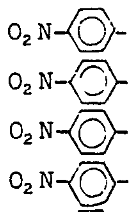 | O | $CH_3$ |
| | O | $C_2H_5$ |
| | $CH_2$ | $CH_3$ |
| | $CH_2$ | $C_2H_5$ |
| 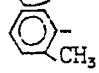 | O | $CH_3$ |
| 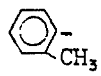 | O | $C_2H_5$ |
| 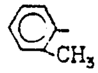 | $CH_2$ | $CH_3$ |
| 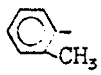 | $CH_2$ | $C_2H_5$ |
| 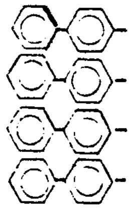 | O | $CH_3$ |
| | O | $C_2H_5$ |
| | $CH_2$ | $CH_3$ |
| | $CH_2$ | $C_2H_5$ |

Verwendet man beispielsweise 1-(4-Chlorphenoxy)-3,3-dimethyl-1-(1,2,4-triazol-1-yl)-butan-2-on und Dimethylsulfat als Ausgangsstoffe, so kann der Reaktionsablauf durch das folgende Formelschema wiedergegeben werden :

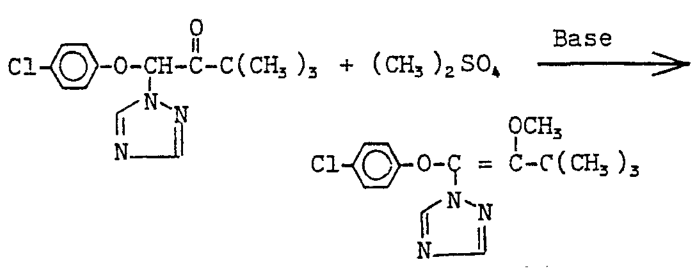


Die bei der Durchführung des erfindungsgemäßen Verfahrens als Ausgangsstoffe zu verwendenden 1,2,4-Triazolyl-ketone sind durch die allgemeine Formel II definiert. In dieser Formel stehen Ar und X vorzugsweise für diejenigen Reste, die bereits im Zusammenhang mit der Beschreibung der erfindungsgemäßen Stoffe der allgemeinen Formel I vorzugsweise für diese Substituenten genannt wurden.

Die 1,2,4-Triazolyl-ketone der allgemeinen Formel II sind bekannt (vergleiche DE-AS 22 01 063 und DE-OS 27 34 426) und können nach den dort angegebenen Verfahren erhalten werden, indem man z.B. entsprechende Halogenketone mit 1,2,4-Triazol in Gegenwart eines Verdünnungsmittels und in Gegenwart eines Säurebinders umsetzt (vergleiche auch die Herstellungsbeispiele).

Die außerdem für das erfindungsgemäße Verfahren als Ausgangsstoffe benötigten Alkylsulfate bzw. -halogenide sind allgemein bekannte Verbindungen der organischen Chemie. Als Beispiele seien genannt: Dimethylsulfat, Diethylsufat, Methylbromid, Methyljodid, Ethylbromid, Ethyljodid, Isopropyljodid und Isobutyljodid.

Für die erfindungsgemäße Umsetzung kommen als Verdünnungsmittel inerte organische Lösungsmittel infrage. Hierzu gehören vorzugsweise aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Formamide, wie Dimethylformamid; sowie Dimethylsulfoxid.

Die erfindungsgemäße Umsetzung wird in Gegenwart einer Base durchgeführt. Hierbei können alle üblichen organischen und insbesondere anorganischen Basen eingesetzt werden, wie vorzugsweise Alkalihydroxide oder Alkalicarbonate, beispielsweise seien Natrium- und Kaliumhydroxid genannt.

Die Reaktionstemperaturen können bei der Durchführung des erfindungsgemäßen Verfahrens in einem größeren Bereich variiert werden. Im allgemeinen arbeitet man zwischen 0 und 100 °C, vorzugsweise zwischen 20 und 80 °C.

Bei der Durchführung des erfindungsgemäßen Verfahrens setzt man vorzugsweise auf 1 Mol 1,2,4-Triazolyl-keton der allgemeinen Formel II 1 bis 2 Mol Alkylsulfat bzw. -halogenid ein. Dabei fallen die Verbindungen der allgemeinen Formel I in Form der geometrischen Isomerengemische an. Die Isolierung der einzelnen geometrischen Isomeren erfolgt nach üblichen Methoden, wie z.B. aufgrund unterschiedlicher Löslichkeit, durch Salzbildung, durch Craig-Verteilung oder durch chromatographische Trennverfahren bzw. durch Kombination dieser Methoden. Eine eindeutige Strukturzuordnung erfolgt aufgrund der $^1$H-NMR-Daten, insbesondere unter Verwendung von Verschiebungsreagenzien. Die durch diese Reagenzien bewirkten Signalverschiebungen sind um so größer, je kleiner der räumliche Abstand zwischen dem komplexierten Zentralatom des Verschiebungsreagenzes und dem betrachteten Proton ist (vergleiche auch die Herstellungsbeispiele).

In einer bevorzugten Ausführungsform wird die erfindungsgemäße Umsetzung in einem Zweiphasensystem, wie beispielsweise wässrige Natron- oder Kalilauge/Toluol oder Methylenchlorid, gegebenenfalls unter Zusatz von 0,1-1 Mol eines Phasen-Transfer-Katalysators, wie beispielsweise Ammonium- oder Phosphoniumverbindungen, beispielsweise seien Benzyl-dodecyl-dimethyl-ammoniumchlorid und Triethyl-benzyl-ammoniumchlorid genannt, durchgeführt (vergleiche auch die Herstellungsbeispiele).

Die erfindungsgemäß herstellbaren Verbindungen der allgemeinen Formel I können in Säureadditions-Salze bzw. Metallsalz-Komplexe überführt werden.

Zur Herstellung von physiologisch verträglichen Säureadditionssalzen der Verbindungen der allgemeinen Formel I kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure. Die Säureadditions-Salze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der allgemeinen Formel I können in einfacher Weise nach üblichen Verfahren erhalten werden, so z.B. durch Lösen des Metallsalzes in Alkohol, z.B. Ethanol und Hinzufügen zur Verbindung der allgemeinen Formel I. Man kann Metallsalz-Komplexe in bekannter Weise, z.B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Getreidemehltau und Gerstenmehltau; von Podosphaera-Arten, wie den Erreger des Apfelmehltaus (Podosphaera leucotricha); sowie von Erysiphe-Arten, wie den Erreger des Gurkenmehltaus (Erysiphe cichoracearum), eingesetzt werden. Hervorzuheben ist, daß die erfindungsgemäßen Wirkstoffe nicht nur eine protektive Wirkung entfalten, sondern teilweise auch

systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glykol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmingen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwiechen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinenzwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

In den nachfolgenden Beispielen werden die nachstehend angegebenen Verbindungen als Vergleichssubstanzen eingesetzt :

A)

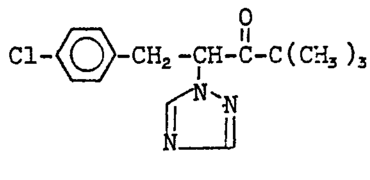

B)

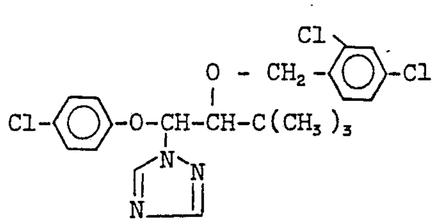

## Beispiel A

Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21-22 °C und einer Luftfeuchtigkeit von 80-90 % wertet man den Besatz der Pflanzen mit Mehltaupusteln aus. Der Befallsgrad wird in Prozent des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Mehltaubefall ist.

Die Testergebnisse sind der nachfolgenden Tabelle zu entnehmen :

### Tabelle A

Sproßbehandlungs-Test/Getreidemehltau/protektiv

| Wirkstoffe | Wirkstoff-konzentration in der Spritz-brühe in Gew.-% | Befall in % der unbe-handelten Kontrolle |
|---|---|---|
| 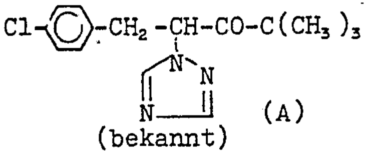 (A) (bekannt) | 0,001 | 72,5 |
| 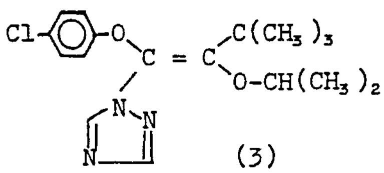 (3) | 0,001 | 26,9 |
| 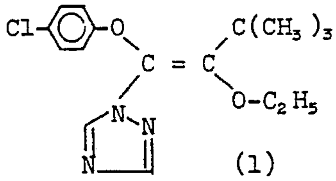 (1) | 0,001 | 25,0 |
| 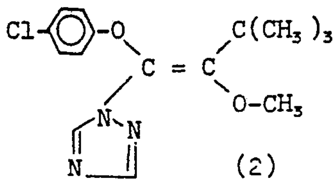 (2) | 0,001 | 0,0 |

## Beispiel B

Gerstenmehltau-Test (Erysiphe graminis var. hordei)/systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21-22 °C und 80-90 % rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0 % keinen Befall und 100 % den gleichen Befallsgrad wie bei der unbehandelten Kontrolle. Der Wirkstoff ist um so wirksamer je geringer der Mehltaubefall ist.

Die Testergebnisse sind der nachfolgenden Tabelle zu entnehmen :

### Tabelle B

Gerstenmehltau-Test (Erysiphe graminis var. hordei)/systemisch

| Wirkstoffe | Wirkstoffkonzentration im Beizmittel in Gew.-% | Beizmittelaufwandmenge in g/kg Saatgut | Befall in % der unbehandelten Kontrolle |
|---|---|---|---|
| 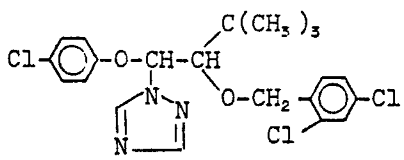 (B) (bekannt) | 25 | 10 | 100 |
| 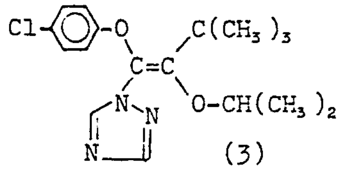 (3) | 25 | 10 | 50,0 |
| 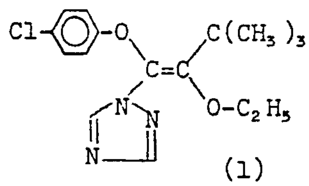 (1) | 25 | 10 | 0,0 |
| 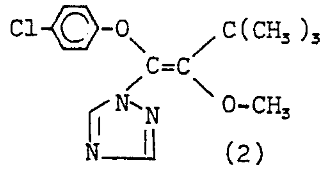 (2) | 25 | 10 | 0,0 |

Tabelle B (Fortsetzung)

Gerstenmehltau-Test (Erysiphe graminis var. hordei)/systemisch

| Wirkstoffe | Wirkstoff-konzentration im Beizmittel in Gew.-% | Beizmittel-aufwandmenge in g/kg Saat-gut | Befall in % der unbehan-delten Kontrolle |
|---|---|---|---|
| 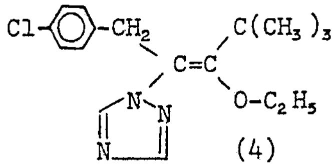 (4) | 25 | 10 | 33,8 |
| 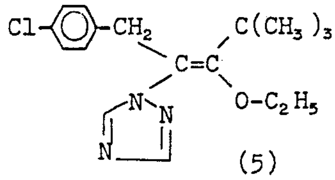 (5) | 25 | 10 | 0,0 |

## Beispiel C

Podosphaera-Test (Apfel)/protektiv

Lösungsmittel : 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 5-Blattstadium befinden, bis zur Tropfnässe. Die Pflanzen verbleiben 24 Stunden bei 20 °C und einer relativen Luftfeuchtigkeit von 70 % im Gewächshaus. Anschließend werden die durch Bestäuben mit Konidien des Apfelmehltauerregers (Podosphaera leucotricha) inokuliert und in ein Gewächshaus mit einer Temperatur von 21 bis 23 °C und einer relativen Luftfeuchtigkeit von ca. 70 % gebracht.

10 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

Die Testergebnisse sind der nachfolgenden Tabelle zu entnehmen :

Tabelle C

Podosphaera-Test (Apfel)/protektiv

| Wirkstoff | Befall in % bei einer Wirkstoffkonzentration von 0,001 % |
|---|---|
| 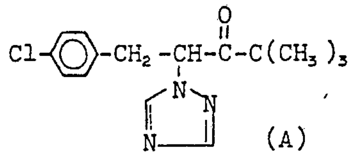 (A) (bekannt) | 62 |

Tabelle C (Fortsetzung)

Podosphaera-Test (Apfel)/protektiv

| Wirkstoff | Befall in % bei einer Wirkstoffkonzentration von 0,001 % |
|---|---|
| 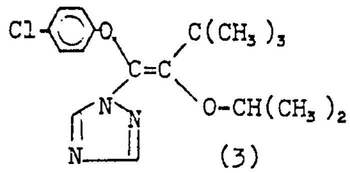 (3) | 25 |
| 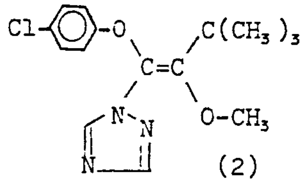 (2) | 22 |
| 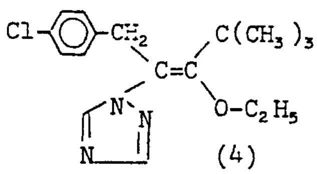 (4) | 32 |

Beispiel D

Erysiphe-Test (Gurken)/protektiv

Lösungsmittel : 4,7 Gewichtsteile Aceton
Emulgator : 0,3 Gewichtsteile Alkyl-aryl-polyglykolether
Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24 °C und bei einer relativen Luftfeuchtigkeit von ca 75 % im Gewächshaus aufgestellt.

Nach 12 Stunden wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Die Testergebnisse sind der nachfolgenden Tabelle zu entnehmen :

Tabelle D

Erysiphe-Test (Gurken)/protektiv

| Wirkstoff | Befall in % bei einer Wirkstoffkonzentration von 0,000 5 % |
|---|---|
| 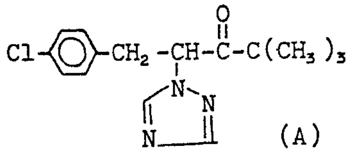 (A) | 62 |

Tabelle D (Fortsetzung)

Erysiphe-Test (Gurken)/protektiv

| Wirkstoff | Befall in % bei einer Wirkstoffkonzentration von 0,000 5 % |
|---|---|
| 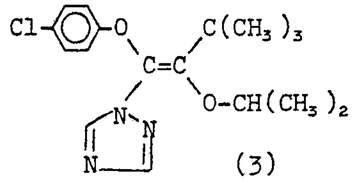 (3) | 12 |
| 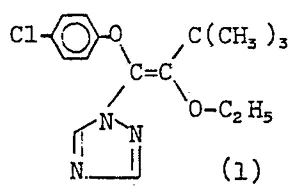 (1) | 19 |

Herstellungsbeispiele

Beispiel 1

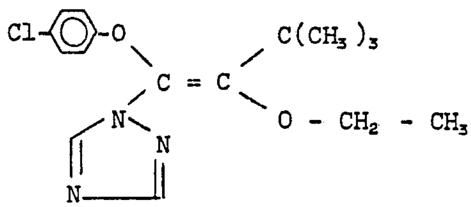

(E)-Form

Zu 29,5 g (0,1 Mol) 1-(4-Chlorphenoxy)-3,3-dimethyl-1-(1,2,4-triazol-1-yl)-butan-2-on, 16 g (0,1 Mol) Ethyljodid und 0,5 g Benzyl-dodecyl-dimethyl-ammoniumchlorid in 50 ml Dimethylsulfoxid werden bei Raumtemperatur langsam 4,4 g (0,11 Mol) Natronlauge, in wenig Wasser gelöst, zugetropft. Die ablaufende Reaktion ist leicht exotherm (bis ca. 50 °C). Danach läßt man 3 Stunden bei Raumtemperatur nachrühren, gibt das Reaktionsgemisch auf Wasser, neutralisiert mit Essigsäure und extrahiert mit Methylenchlorid. Die organische Phase wird mit Wasser ausgeschüttelt, über Natriumsulfat getrocknet und eingeengt. Nach Trennung durch Craig-Verteilung erhält man 6,0 g (22 % der Theorie) 1-(4-Chlorphenoxy)-3,3-dimethyl-2-ethoxy-1-(1,2,4-triazol-1-yl)-1-buten als Isomerengemisch (Z/E = 1/3). Durch präparative HPLC erhält man die (Z)- und die (E)-Form des 1-(4-Chlorphenoxy)-3,3-dimethyl-2-ethoxy-1-(1,2,4-triazol-1-yl)-1-butens, wobei die (E)-Form einen Schmelzpunkt von 57-62 °C besitzt.

Die Strukturzuordnung der beiden Isomeren erfolgt mittels $^1H$-NMR-Spektroskopie unter Verwendung von $Eu(TFC)_3$ = Europium-tris-[3-trifluormethylhydroxymethylen)-d-camphorato] als Verschiebungsreagenz. Die Messungen werden in $CDCl_3$ durchgeführt. Die Ergebnisse gehen aus der nachfolgenden Tabelle hervor :

(Siehe die Tabelle, Seite 12)

Tabelle : Shiftwerte und Verschiebungsdifferenzen der Resonanzsignale mit und ohne Verschiebungsreagenz (VR)

| Fragment: | (Z)-Isomeres | | | (E)-Isomeres | | |
|---|---|---|---|---|---|---|
| | δ (ppm) ohne VR: | δ (ppm) mit VR: | ΔS | δ (ppm) ohne VR: | δ (ppm) mit VR: | ΔS |
| $C(CH_3)_3$ | 1,00 | 2,77 | 1,77 | 1,15 | 2,16 | 1,01 |
| $CH_3$ | 1,22 | 1,80 | 0,58 | 1,02 | 2,70 | 1,68 |
| $CH_2$ | 4,02 | 5,10 | 1,08 | 3,33 | 6,45 | 3,12 |
| Triaz.-H | 7,94<br>8,10 | > 12 | > 4 | 7,91<br>8,31 | >12 | > 4 |
| arom. H | 7,02<br>7,25 | 7,80<br>8,60 | 0,78<br>1,35 | 6,92<br>7,19 | 7,58<br>8,46 | 0,66<br>1,27 |

Erläuterungen :
« Craig-Verteilung » = Gegenstromverteilung
HPLC = Hochdruckflüssigkeitschromatographie
Zu $Eu(TFC)_3$ siehe Merck, Kontakte, Heft 2/1978, S. 9.

Herstellung des Ausgangsproduktes

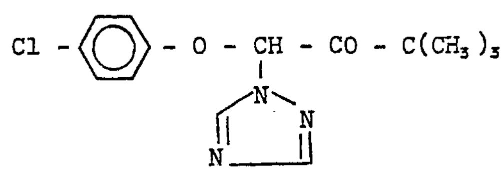

418 g (6,6 Mol) 1,2,4-Triazol werden in 3 000 ml Aceton gelöst. Hierzu werden 934 g (7,2 Mol) wasserfreies, pulverisiertes Kaliumcarbonat gegeben, die Suspension zum Sieden erhitzt und eine Lösung von 1 565 g (6 Mol) 1-(4-Chlorphenoxy)-1-chlor-3,3-dimethyl-butan-2-on in 1 500 ml Aceton so zugetropft, daß die Mischung ohne Beheizung center Rückfluß siedet. Nach beendeter Zugabe wird zur Vervollständigung der Reaktion 15 Stunden unter Rückfluß erhitzt ; anschließend wird der erhaltene Niederschlag abfiltriert, mit Aceton gewaschen und verworfen. Das Filtrat wird im Wasserstrahlvakuum vom Lösungsmittel befreit, der Rückstand in 3 000 ml Toluol aufgenommen und einmal mit einer Lösung von 100 g 37 %-iger Salzsäure in 2 000 ml Wasser gewaschen. Die wässrige Phase wird abgetrennt und verworfen ; die organische Phase wird mit 5 000 ml Wasser gewaschen und — nach Zugabe von weiteren 4 000 ml Toluol — mit einer Lösung von 145 g Natriumhydroxid in 3 500 ml Wasser 6 Stunden bei Raumtemperatur gerührt. Danach wird die organische Phase abgetrennt, mit Wasser neutral gewaschen und im Wasserstrahlvakuum vom Lösungsmittel befreit. Man erhält 1 535 g (87 % der Theorie) 1-(4-Chlorphenoxy)-3,3-dimethyl-1-(1,2,4-triazol-1-yl)-butan-2-on vom Schmelzpunkt 75-76 °C.

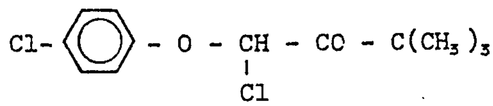

771 g (6 Mol) 4-Chorphenol werden in 3 600 ml Aceton gelöst. Man trägt 3 g wasserfreies Natriumjodid sowie 910 g (6,6 Mol) wasserfreies, pulverisiertes Kaliumcarbonat ein und läßt unter Rückfluß 895 g (6,3 Mol) 94,6 %-iges Monochlorpinakolin zutropfen. Nach 20-stündigem Rühren bei Rückflußtemperatur wird der Niederschlag abfiltriert, mit Aceton gewaschen und verworfen. Das Filtrat wird im Wasserstrahlvakuum vom Lösungsmittel befreit. Der resultierende weiße Rückstand wird mit 3 000 ml Tetrachlorkohlenstoff aufgenommen und auf 60 °C erwärmt. Zu dieser Lösung werden 891 g (6,6 Mol) Sulfurylchlorid ohne weitere Erwärmung so zugetropft, daß eine stetige Gasentwicklung erfolgt. Nach beendeter Zugabe wird 15 Stunden unter Rückfluß erhitzt. Schließlich wird das Lösungsmittel im

Wasserstrahlvakuum abdestilliert. Man erhält in quantitativer Ausbeute 1 565 g 1-(4-Chlorphenoxy)-1-chlor-3,3-dimethyl-butan-2-on, das ohne weitere Reinigung zur oben beschriebenen Umsetzung verwendet werden kann.

Beispiel 2

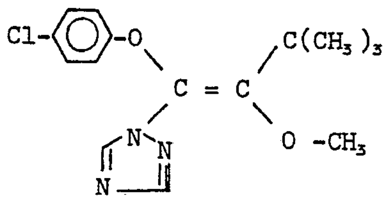

(E)-Form

Zu 146,5 g (0,5 Mol) 1-(4-Chlorphenoxy)-3,3-dimethyl-1-(1,2,4-triazol-1-yl)-butan-2-on in 250 ml Dimethylsulfoxid werden 30 g Kaliumhydroxid, in wenig Wasser gelöst, zugetropft. Man läßt 1 Stunde nachrühren und tropft anschließend 65 g (0,51 Mol) Dimethylsulfat zu. Dabei wird die Temperatur der Reaktionsmischung mittels Eisbad auf ca. 40 °C gehalten. Man läßt 2 Stunden nachrühren, versetzt mit Wasser, saugt den kristallinen Niederschlag ab und kristallisiert aus Petrolether um. Man erhält 51 g (33 % der Theorie) (E)-1-(4-Chlorphenoxy)-3,3-dimethyl-2-methoxy-1-(1,2,4-triazol-1-yl)-1-buten vom Schmelzpunkt 134-138 °C.

In entsprechender Weise werden die nachfolgenden Beispiele der allgemeinen Formel I

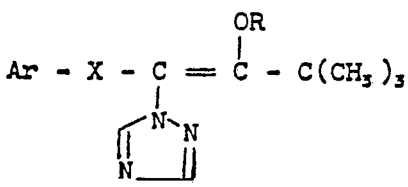

(I)

erhalten :

| Bsp.Nr. | Ar | X | R | Schmelzpunkt(°C) |
|---|---|---|---|---|
| 3 | 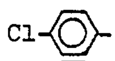 | O | i-$C_3H_7$ | 129-32(E-Form) |
| 4 | 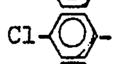 | $CH_2$ | $C_2H_5$ | 103-05(Z-Form) |
| 5 | 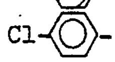 | $CH_2$ | $C_2H_5$ | $n_D^{22}$=1,5370 (E-Form) |

**Ansprüche**

1. 1,2,4-Triazolyl-enolether der allgemeinen Formel I

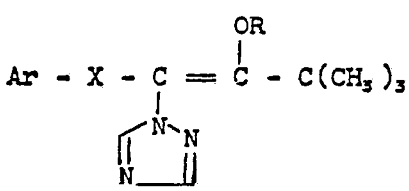

(I)

in welcher

Ar für Phenyl und Naphthyl steht, wobei diese Reste substituiert sein können durch Halogen, Alkyl mit 1 bis 6 und Alkoxy mit 1 bis 4 Kohlenstoffatomen, Halogenalkyl mit 1 bis 4 Kohlenstoff- und 1 bis 5 Halogenatomen, ferner durch Nitro, Cyano und schließlich durch gegebenenfalls halogensubstituiertes Phenyl,

R für Alkyl mit 1 bis 4 Kohlenstoffatomen steht und

X für Sauerstoff oder für die Methylengruppe steht, sowie deren Säure- und Metallsalz-Additionsverbindungen.

2. Verfahren zur Herstellung von 1,2,4-Triazolyl-enolethern der allgemeinen Formel I

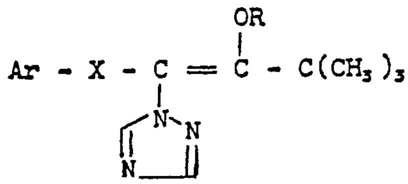

(I)

in welcher

Ar für Phenyl und Naphthyl steht, wobei diese Reste substituiert sein können durch Halogen, Alkyl mit 1 bis 6 und Alkoxy mit 1 bis 4 Kohlenstoffatomen, Halogenalkyl mit 1 bis 4 Kohlenstoff- und 1 bis 5 Halogenatomen, ferner durch Nitro, Cyano und schließlich durch gegebenenfalls halogensubstituiertes Phenyl,

R für Alkyl mit 1 bis 4 Kohlenstoffatomen steht und

X für Sauerstoff oder für die Methylengruppe steht, dadurch gekennzeichnet, daß man 1,2,4-Triazolyl-ketone der allgemeinen Formel II

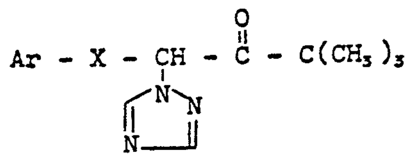

(II)

in welcher

Ar und X die oben angegebene Bedeutung haben, mit entsprechenden Alkylsulfaten bzw.-halogeniden in Gegenwart einer Base und in Gegenwart eines organischen Verdünnungsmittels, oder in einem wäßrigorganischen Zweiphasensystem in Gegenwart eines Phasentransferkatalysators umsetzt, und gegebenenfalls anschließend eine Säure oder ein Metallsalz addiert.

3. Fungizide Mittel für den Pflanzenschutz, gekennzeichnet durch einen Gehalt an mindestens einem 1,2,4-Triazolyl-enolether der allgemeinen Formel I in Ansprüchen 1 und 2.

4. Verwendung von 1,2,4-Triazolyl-enolethern der allgemeinen Formel I in Ansprüchen 1 und 2 zur Bekämpfung von Pilzen im Pflanzenschutz.

5. Verfahren zur Herstellung von fungiziden Mitteln für den Pflanzenschutz, dadurch gekennzeichnet, daß man 1,2,4-Triazolyl-enolether der allgemeinen Formel I in Ansprüchen 1 und 2 mit Streckmitteln und/oder oberflächenaktiven Mitteln vermischt.

**Claims**

1. 1,2,4-triazolyl-enol ethers of the general formula I

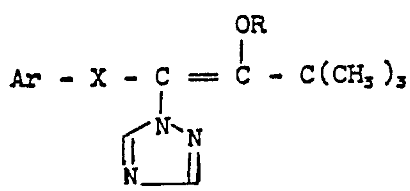

(I)

in which

Ar represents phenyl and naphthyl, it being possible for these radicals to be substituted by halogen, alkyl with 1 to 6 carbon atoms and alkoxy with 1 to 4 carbon atoms, halogenoalkyl with 1 to 4 carbon atoms and 1 to 5 halogen atoms, furthermore by nitro or cyano and finally by optionally halogen-substituted phenyl,

R represents alkyl with 1 to 4 carbon atoms and

X represents oxygen or the methylene group, and acid addition compounds and metal salt addition compounds thereof.

2. Process for the preparation of 1,2,4-triazolyl-enol ethers of the general formula I

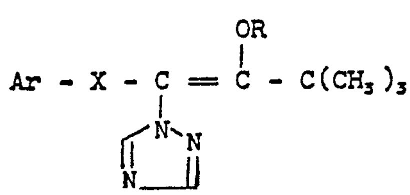

(I)

in which

Ar represents phenyl and naphthyl, it being possible for these radicals to be substituted by halogen, alkyl with 1 to 6 carbon atoms and alkoxy with 1 to 4 carbon atoms, halogenoalkyl with 1 to 4 carbon atoms and 1 to 5 halogen atoms, furthermore by nitro or cyano and finally by optionally halogen-substituted phenyl,

R represents alkyl with 1 to 4 carbon atoms and

X represents oxygen or the methylene group, characterised in that 1,2,4-triazolyl-ketones of the general formula II

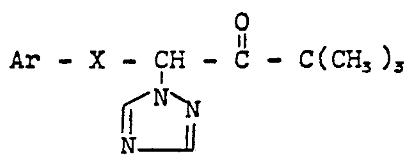

(II)

in which

Ar and X have the meaning indicated above, are reacted with corresponding alkyl sulphates or halides in the presence of a base and in the presence of an organic diluent, or in an aqueous-organic two-phase system in the presence of a phase-transfer catalyst, and optionally an acid or a metal salt is then added on.

3. Fungicidal agents for plant protection, characterised in that they contain at least one 1,2,4-triazolyl-enol ether of the general formula I in Claims 1 and 2.

4. Use of 1,2,4-triazolyl-enol ethers of the general formula I in Claims 1 and 2 for combatintg fungi in plant protection.

5. Process for the preparation of fungicidal agents for plant protection, characterised in that 1,2,4-triazolyl-enol ethers of the general formula I in Claims 1 and 2 are mixed with extenders and/or surface-active agents.

## Revendications

1. Ethers de 1,2,4-triazolyl-énols de formule générale I

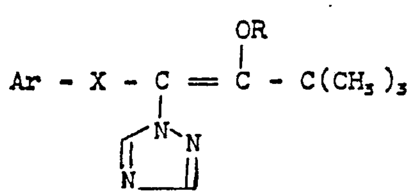

(I)

dans laquelle

Ar représente un groupe phényle et naphtyle, ces restes pouvant être substitués par des halogènes, des groupes alkyle en $C_1$-$C_6$ et alcoxy en $C_1$-$C_4$, halogénoalkyle contenant de 1 à 4 atomes de carbone et de 1 à 5 atomes d'halogènes, ou encore par des groupes nitro, cyano et finalement par un groupe phényle éventuellement substitué par des halogènes,

R représente un groupe alkyle en $C_1$-$C_4$ et

X représente l'oxygène ou le groupe méthylène, ainsi que leurs composés formés par addition avec des acides et des sels métalliques.

2. Procédé de préparation des éthers de 1,2,4-triazolyl-énols de formule générale I

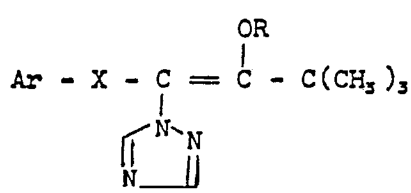

(I)

dans laquelle

Ar représente un groupe phényle et naphtyle, ces restes pouvant être substitués par des halogènes, des groupes alkyle en $C_1$-$C_6$ et alcoxy en $C_1$-$C_4$, halogénoalkyle contenant de 1 à 4 atomes de carbone et de 1 à 5 atomes d'halogènes, ou encore par des groupes nitro, cyano et finalement par un groupe phényle éventuellement substitué par des halogènes,

R représente un groupe alkyle en $C_1$-$C_4$ et

X représente l'oxygène ou le groupe méthylène, caractérisé en ce que l'on fait réagir des 1,2,4-triazolyl-cétones de formule générale II

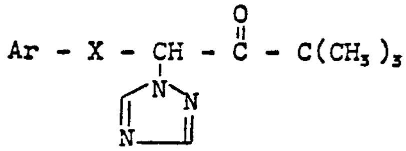

dans laquelle

Ar et X ont les significations indiquées ci-dessus, avec des sulfates ou halogénures d'alkyle correspondants en présence d'une base et en présence d'un diluant organique, ou dans un système à deux phases hydro-organique en présence d'un catalyseur à transfert de phases, après quoi, le cas échéant, on fixe un acide ou un sel métallique par addition.

3. Produits fongicides pour la protection des végétaux, caractérisés en ce qu'ils contiennent au moins un éther de 1,2,4-triazolyl-énol de formule générale I selon les revendications 1 et 2.

4. Utilisation des éthers de 1,2,4-triazolyl-énols de formule générale I selon les revendications 1 et 2 pour combattre les mycètes dans la protection des végétaux.

5. Procédé de préparation de produits fongicides pour la protection des végétaux, caractérisé en ce que l'on mélange des éthers de 1,2,4-triazoly-énols de formule générale I selon les revendications 1 et 2 avec des diluants et /ou des agents tensio-actifs.